(19) Europäisches Patentamt
European Patent Office
Office européen des brevets

(11) EP 1 799 186 B1

(12) EUROPÄISCHE PATENTSCHRIFT

(45) Veröffentlichungstag und Bekanntmachung des Hinweises auf die Patenterteilung:
**18.06.2008 Patentblatt 2008/25**

(21) Anmeldenummer: **05777078.6**

(22) Anmeldetag: **19.08.2005**

(51) Int Cl.:
***A61K 8/49*** *(2006.01)* ***A61K 31/4425*** *(2006.01)*
***A61Q 11/00*** *(2006.01)* ***A61K 9/00*** *(2006.01)*

(86) Internationale Anmeldenummer:
**PCT/EP2005/008973**

(87) Internationale Veröffentlichungsnummer:
**WO 2006/039961 (20.04.2006 Gazette 2006/16)**

# (54) OCTENIDINHALTIGE LUTSCHTABLETTEN GEGEN ENTZÜNDLICHE ERKRANKUNGEN DES MUND- UND RACHENRAUMS

OCTENIDINE-CONTAINING LOZENGES USED AGAINST INFLAMMATORY ORAL AND PHARYNGEAL DISEASES

COMPRIMES A SUCER CONTENANT DE L'OCTENIDINE, CONTRE LES MALADIES INFLAMMATOIRES DE LA CAVITE BUCCOPHARYNGEE

(84) Benannte Vertragsstaaten:
**AT BE BG CH CY CZ DE DK EE ES FI FR GB GR HU IE IS IT LI LT LU LV MC NL PL PT RO SE SI SK TR**
Benannte Erstreckungsstaaten:
**AL BA HR MK YU**

(30) Priorität: **12.10.2004 DE 102004049798**
**28.10.2004 DE 102004052308**

(43) Veröffentlichungstag der Anmeldung:
**27.06.2007 Patentblatt 2007/26**

(73) Patentinhaber: **Divapharma GmbH**
**12277 Berlin (DE)**

(72) Erfinder: **MIETHING, Holger**
**12107 Berlin (DE)**

(74) Vertreter: **Strehlke, Ingo Kurt**
**Gesthuysen, von Rohr & Eggert**
**Postfach 10 13 54**
**45013 Essen (DE)**

(56) Entgegenhaltungen:
**EP-A- 0 144 960**
**WO-A-87/05501**
**WO-A-93/16681**
**WO-A-03/067988**
**DE-A1- 10 026 716**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die vorliegende Erfindung betrifft eine pharmazeutische Zusammensetzung in Form einer festen Dosierung zum Lutschen, insbesondere in Form einer Lutschtablette, zur topischen Behandlung von entzündlichen Erkrankungen des Mund- und Rachenraums, wobei die pharmazeutische Zusammensetzung eine therapeutisch wirksame Menge an Octenidin, insbesondere in Form des Octenidindihydrochlorids, enthält.

Des weiteren betrifft die vorliegende Erfindung die Verwendung von Octenidin, insbesondere in Form des Octenidindihydrochlorids, zur topischen Behandlung von entzündlichen Erkrankungen des Mund- und Rachenraums bzw. zur Herstellung eines Arzneimittels zur topischen Behandlung von entzündlichen Erkrankungen des Mund- und Rachenraums.

Entzündliche Erkrankungen des Mund- und Rachenraums, d. h. Entzündungen im Bereich der Mundhöhle und des Hals-/Rachenraums, treten oftmals als Begleiterscheinung von Erkältungserkrankungen und grippalen Infekten, aber auch als eigenständige Erkrankungen auf. Solche entzündlichen Prozesse des Mund- und Rachenraums sind oftmals mit einer für den betroffenen Patienten unangenehmen Schmerzsymptomatik verbunden. Nicht beschränkende Beispiele für derartige entzündliche Erkrankungen des Hals-/Rachenraums sind Halsentzündungen, insbesondere Angina, Entzündungen des Kehlkopfs (Laryngitis), Entzündungen der Rachenschleimhaut (Pharyngitis) und Entzündungen der Rachenmandeln (Tonsillitis). Auch Entzündungen im Bereich der Mundhöhle, wie z. B. Stomatitis, Gingivitis, Mundschleimhautläsionen und dergleichen, gehören zu den entzündlichen Erkrankungen des Mund-/Rachenraums. Für weitere Einzelheiten zu den vorgenannten Erkrankungen kann beispielsweise auf Roche-Lexikon Medizin, 3. Auflage, 1993, Urban & Schwarzenberg, München/Wien/Baltimore, sowie auf Pschyrembel, Medizinisches Wörterbuch, 257. Auflage, 1993, Nikol Verlagsgesellschaft mbH, Hamburg, verwiesen werden. Der Begriff "entzündliche Erkrankungen des Mund- und Rachenraums" ist somit im Rahmen der vorliegenden Erfindung sehr weit zu verstehen und umfaßt sämtliche entzündlichen Erkrankungen, welche im Bereich der Mundhöhle, des Rachens und des Halsraums auftreten können.

Die Behandlung entzündlicher Erkrankungen des Mund- und Rachenraums erfolgt - in Abhängigkeit von der Schwere des Krankheitsbildes - im allgemeinen durch topische und gegebenenfalls zusätzliche systemische Therapie in schwerwiegenderen Fällen. Während in schwereren Fällen systemisch Antibiotika verabreicht werden, kommen unterstützend und in leichteren Fällen unter Umständen alleinig zur topischen, symptomatischen Behandlung oftmals Antiseptika und entzündungshemmende Stoffe (so z. B. Antiphlogistika, antiinflammatorisch wirkende Mittel, Lokalantibiotika etc.) zum Einsatz, welche beispielsweise in Form von Rachenspülungen, Sprays oder Lutschtabletten verabreicht werden können.

Als Wirkstoff zur topischen Behandlung von entzündlichen Prozessen des Mund- und Rachenraums hat sich insbesondere 1-Hexadecylpyridiniumchlorid (internationaler Freiname: "Cetylpyridiniumchlorid" (CPC)) bewährt. Hierbei handelt es sich um eine quaternäre Ammoniumverbindung mit bakterizider und fungizider Wirkung, welche unter anderem in Lutschtabletten zur Anwendung kommt. Nachteilig bei diesem Wirkstoff ist die Tatsache, daß bei hoher Dosierung und übermäßigem Verzehr Magen-Darm-Beschwerden, Atemnot sowie eine vermehrte Methämoglobinbildung, insbesondere bei Kindern, auftreten kann.

Des weiteren hat sich als topisches Antiseptikum bzw. Desinfektionsmittel für die Mund-, Hals- und Rachenschleimhaut auch 1,3-Bis(2-ethylhexyl)-hexahydro-5-methyl-5-pyridinamin (internationaler Freiname: "Hexetidin") bewährt, welches beispielsweise als Spray oder in Form von Spül- bzw. Gurgellösungen appliziert werden kann. Bei längerer Anwendung und starker Dosierung kann es auch hier zu Magen-Darm-Beschwerden und darüber hinaus zu Geschmacksirritationen kommen. Die Applikationsform des Sprays oder der Spül- und Gurgellösungen ist insofern nachteilig, als eine genaue Dosierung nicht ohne weiteres gegeben ist. Spül- und Gurgellösungen sind zudem nicht überall und jederzeit anwendbar. Außerdem haben Lösungen den Nachteil einer relativ begrenzten Haltbarkeit des Wirkstoffes.

Des weiteren kommen Wirkstoffe natürlichen Ursprungs zum Einsatz, so z. B. sogenannte Schleimdrogen oder deren Extrakte, wie z. B. Isländisches Moos *(Lichen islandicus).* Derartige Präparate bieten aber nicht immer den gewünschten Therapieerfolg.

Der vorliegenden Erfindung liegt daher die Aufgabe zugrunde, eine pharmazeutische Zusammensetzung bereitzustellen, welche sich zur effizienten topischen Behandlung von entzündlichen Erkrankungen des Mund- und Rachenraums (d. h. also von entzündlichen Erkrankungen des Hals-/Rachenraums und der Mundhöhle) eignet und insbesondere die zuvor geschilderten Nachteile des Standes der Technik zumindest weitgehend vermeidet oder wenigstens abschwächt.

Die Anmelderin hat nun überraschenderweise herausgefunden, daß das zuvor geschilderte Problem dadurch gelöst werden kann, daß man eine pharmazeutische Zubereitung in Form einer festen Dosierung zum Lutschen, insbesondere in Form einer Lutschtablette, auf Basis von Octenidin, insbesondere in Form des Octenidindihydrochlorids, gegebenenfalls zusammen mit mindestens einem zur topischen Anwendung geeigneten Lokalanästhetikum, formuliert. Eine solche pharmazeutische Zusammensetzung eignet sich insbesondere zur Herstellung von Lutschtabletten, vorzugsweise auf Hartkaramellenbasis, welche bei entzündlichen Erkrankungen des Mund- und Rachenraums zur topischen

Anwendung gelangen können.

Gegenstand der vorliegenden Erfindung ist somit eine pharmazeutische Zusammensetzung in fester Dosierungsform zum Lutschen, insbesondere in Form einer Lutschtablette, zur topischen Behandlung von entzündlichen Erkrankungen des Mund- und Rachenraums, wobei die Zusammensetzung eine therapeutisch wirksame Menge an Octenidin, vorzugsweise in Form des Octenidindihydrochlorids, enthält. Dabei ist der Begriff "topische Behandlung" sehr weit zu verstehen und umfaßt sowohl die prophylaktische topische Behandlung, insbesondere zu Desinfektionszwecken in bezug auf den Mund- und Rachenraum, sowie die therapeutische bzw. kurative topische Behandlung im Fall des akuten entzündlichen Stadiums der vorgenannten Erkrankungen des Mund- und Rachenraums.

Die Anmelderin hat nun überraschenderweise festgestellt, daß Octenidin, insbesondere in Form des Octenidindihydrochlorids, sich ausgezeichnet zur topischen Behandlung von entzündlichen Erkrankungen des Mund- und Rachenraums eignet, wenn es in Form einer festen Dosierung zum Lutschen, insbesondere als Lutschtablette, bevorzugt in Form einer sogenannten Hartkaramelle, appliziert wird derart, daß eine therapeutisch wirksame Menge Octenidin, vorzugsweise Octenidindihydrochlorid, beim Lutschen freigesetzt wird, wenn die feste Dosierungsform zum Lutschen im Mund- und Rachenraum eines Patienten verabreicht und gelutscht wird.

Octenidindihydrochlorid ist der internationale Freiname für das bakteriostatisch wirkende Desinfektionsmittel 1,1'-(1,10-Decandiyl)bis[4-(octylamino)-pyridinium]-dichlorid bzw. 1,1'-Decamethylen[(1,4-dihydro-4-octylimimo)pyridinium]dichlorid $C_{36}H_{64}N_4Cl_2$. Diese Verbindung kann durch die folgende Strukturformel dargestellt werden:

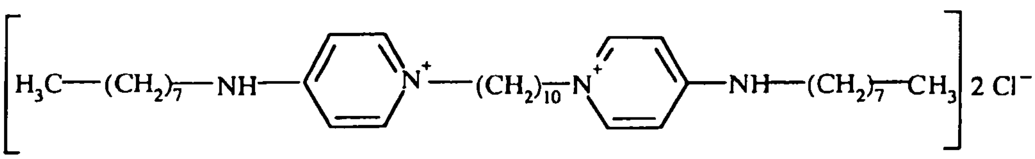

Das in Wasser unlösliche Octenidindihydrochlorid gehört in die Gruppe der quartären Ammoniumverbindungen und enthält im Unterschied zu den anderen Substanzen dieser Verbindungsklasse, wie z. B. Benzalkoniumchlorid und Cetylpyridiniumchlorid, zwei kationenaktive Zentren. Eine ähnliche Struktur hat Dequaliniumchlorid. Octenidindihydrochlorid ist zugelassen unter anderem zur Hautdesinfektion vor Operationen, zur einmaligen Wund- und Nahtversorgung sowie zur hygienischen und chirurgischen Handdesinfektion. Die Wirkung tritt rasch ein und hält lange an.

Obwohl der Wirkstoff als solcher bereits 1977 zum Patent angemeldet worden ist (DE 27 08 331 C2) und seitdem zahlreiche Anwendungen des Octenidins bekannt geworden sind und sich in der Medizin auch etabliert haben, ist das pharmazeutische Potential von Octenidin in bezug auf die topische Behandlung von entzündlichen Erkrankungen des Mund- und Rachenraums bislang noch nicht vollständig erkannt und folglich noch nicht zur optimalen Anwendung gebracht worden. Zwar gibt es zahlreiche Marktprodukte, die Octenidin zu Zwecken der Wunddesinfektion zur Anwendung bringen (vgl. das Marktprodukt Octenisept® der Schülke & Mayr GmbH), jedoch kommt hier das Octenidin meistens in Form wäßriger, alkoholischer oder wäßrig-alkoholischer Lösungen zum Einsatz.

Ferner sind aus der WO 03/067988 A1, der EP 0 144 960 A2, der WO 87/05501 A1, der WO 93/16681 A1 und der DE 100 26 716 A1 verschiedene Octenidin enthaltende Zusammensetzungen in Form von Lösungen, Zahnpasten und Mundspüllösungen bekannt. Octenidinhaltige Zubereitungen in Form einer festen Dosierung zum Lutschen sind dagegen dort nicht beschrieben.

Das pharmazeutische Wirkpotential des Octenidins in bezug auf die topische Behandlung entzündlicher Erkrankungen des Mund- und Rachenraums ist somit bislang unerkannt geblieben, insbesondere in fester Dosierungsform zum Lutschen. Diese Erkenntnis geht erst auf die Anmelderin zurück. Denn erst die Anmelderin hat den Wirkstoff Octenidin in Form einer festen galenischen Zubereitung zum Lutschen, insbesondere in Form einer Lutschtablette, formuliert, so daß auf diese Weise das Wirkpotential des Octenidins, insbesondere des Octenidindihydrochlorids, in bezug auf seine bakterizide, fungizide und viruzide Wirkung zum Einsatz kommt, um zur topischen Behandlung von entzündlichen Erkrankungen des Mund- und Rachenraums eingesetzt zu werden. Hierin muß das besondere Verdienst der vorliegenden Erfindung gesehen werden.

Die Menge an Octenidin, insbesondere Octenidindihydrochlorid, in der erfindungsgemäßen pharmazeutischen Zusammensetzung kann in weiten Bereich variieren. Im allgemeinen liegt die Menge an Octenidin, insbesondere Octenidindihydrochlorid, im Bereich von 0,01 bis 5 Gew.-%, insbesondere 0,025 bis 3 Gew.-%, vorzugsweise 0,05 bis 2,5 Gew.-%, bezogen auf die erfindungsgemäße pharmazeutische Zusammensetzung. Dennoch ist es möglich, von den vorgenannten Mengenbereichen abzuweichen, insbesondere wenn dies anwendungsbedingt vorteilhaft oder erforderlich ist.

Die erfindungsgemäße pharmazeutische Zusammensetzung eignet sich insbesondere zur temporären alleinigen oder unterstützenden Behandlung bei Entzündungen des Mund- und Rachenraums, da die erfindungsgemäße

pharmazeutische Zusammensetzung entzündungshemmend bzw. entzündungslindernd wirkt und auf diese Weise auch eine effiziente Schmerzlinderung bewirkt.

Der Begriff "pharmazeutische Zusammensetzung" ist sehr weit zu verstehen und umfaßt jede Art von möglicher pharmazeutische Zusammensetzung oder galenischer Zubereitung, insbesondere Arzneimittel bzw. Pharmaka als solche, aber auch Medizinprodukte, homöopathische Mittel etc.

Die therapeutische Wirksamkeit der erfindungsgemäßen pharmazeutischen Zusammensetzung kann noch dadurch gesteigert werden, daß ihr mindestens ein Lokalanästhetikum zugesetzt wird. Die Menge an Lokalanästhetikum in der erfindungsgemäßen pharmazeutischen Zusammensetzung kann in weiten Bereichen variieren. Vorteilhafterweise liegt sie im Bereich von 0,01 bis 5 Gew.-%, insbesondere 0,05 bis 4 Gew.-%, vorzugsweise 0,1 bis 2,5 Gew.-%, besonders bevorzugt 0,2 bis 0,6 Gew.-%, bezogen auf die erfindungsgemäße pharmazeutische Zusammensetzung. Dennoch kann es anwendungsbedingt oder einzelfallabhängig vorteilhaft bzw. erforderlich sein, von den vorgenannten Mengenbereichen abzuweichen.

Was das erfindungsgemäß eingesetzte Lokalanästhetikum anbelangt, so können grundsätzlich alle zur topischen Anwendung geeigneten Lokalanästhetika zur Anwendung kommen.

Lokalanästhetika heben reversibel und örtlich begrenzt die Erregbarkeit der schmerzvermittelnden sensiblen Endorgane und das Leitungsvermögen der sensiblen Nervenfasern auf. Als Folge davon wird die Schmerzempfindung ohne Beeinträchtigung des Bewußtseins vorübergehend ausgeschaltet. Die Wirkung von Lokalanästhetika auf die sensorischen Nervenendigungen ist nicht spezifisch, die verschiedenen erregbaren Strukturen sind allerdings unterschiedlich empfindlich. Daß z. B. die motorischen Funktionen bei den für Lokalanästhetika üblichen Dosierungen nicht ausfallen, beruht vor allem darauf, daß die motorischen Nervenfasern einen größeren Durchmesser als die sensorischen, für die "Schmerzleitung" wichtigen Nervenfasern besitzen. Für weitergehende Einzelheiten zu Lokalanästhetika kann beispielsweise verwiesen werden auf E. Mutschler et al. "Mutschler Arzneimittelwirkungen - Lehrbuch der Pharmakologie und Toxikologie", 8. Auflage, Wissenschaftliche Verlagsgesellschaft mbH Stuttgart, 2001, Seiten 267 ff., und Römpp Chemie-Lexikon, 10. Auflage, Band 3, Georg Thieme Verlag, Stuttgart/New York, 1997, Seite 2442, Stichwort: "Lokalanästhetika", sowie die dort jeweils referierte Literatur.

Erfindungsgemäß geeignete Lokalanästhetika sind beispielsweise Lokalanästhetika auf Basis organischer Säureester oder Säureamide. Vorzugsweise wird ein Lokalanästhetikum vom Estertyp gewählt. Beispiele für Lokalanästhetika vom Estertyp sind Benzocain (Ethoform), Procain und Tetracain. Lokalanästhetika vom Amidtyp sind insbesondere Lidocain, Etidocain, Pridocain, Mepivacain, Bupivacain, S-Ropivacain und Articain.

Ganz besonders bevorzugt wird als Lokalanästhetikum in der erfindungsgemäßen pharmazeutischen Zusammensetzung Benzocain eingesetzt. Denn die Anmelderin hat überraschenderweise herausgefunden, daß im Rahmen der erfindungsgemäßen pharmazeutischen Zusammensetzung Benzocain zusammen mit Octenidin bzw. Octenidindihydrochlodrid die beste Wirkung entfaltet. Ohne sich auf eine bestimmte Theorie festlegen zu wollen, läßt sich die besonders gute Wirkung des Benzocains, eines neutralen nichtionisierbaren primären Amins, möglicherweise darauf zurückführen, daß die durch das Benzocain bewirkte Blockade der Erregungsleitung darauf beruht, daß das normale Membrangefüge durch die "Einlagerung" des Benzocains in die Lipidphase so desintegriert wird, daß es dadurch indirekt zu einer Blockade des Natriumkanals kommt, d. h. dieser Wirkmechanismus von dem anderer Lokalanästhetika des klassischen Typs (z. B. auf Basis sekundärer oder tertiärer Amine) abweicht. Dieser abweichende Wirkmechanismus ist insofern von Bedeutung, als entzündetes Gewebe infolge einer lokalen Lactatacidose einen niedrigeren pH-Wert gegenüber normalem Gewebe aufweist. Klassische Lokalanästhetika vom Typ sekundärer oder tertiärer Amine liegen dann bei einem pH-Wert von 7,4 und weniger in der Wasserphase größtenteils ionisiert vor, wodurch das Penetrationsvermögen erniedrigt wird und - im Gegensatz zu dem nichtionisierbaren Benzocain - ein Wirkungsverlust eintreten kann. Daher ist eine pharmazeutische Kombination von Octenidin, insbesondere Octenidindihydrochlorid, mit Benzocain besonders bevorzugt. Aber auch die Kombination von Octenidin, insbesondere Octenidindihydrochlorid, mit anderen Lokalanästhetika (z. B. Lidocain) entfaltet ein effizientes Wirkspektrum.

Darüber hinaus kann es gegebenenfalls vorteilhaft sein, wenn die erfindungsgemäße pharmazeutische Zusammensetzung außerdem mindestens eine Schleimdroge und/oder deren Extrakt enthält. Im Rahmen der vorliegenden Erfindung können eine Vielzahl von Schleimdrogen bzw. deren Extrakte zur Anwendung kommen. Erfindungsgemäß geeignete Schleimdrogen können insbesondere ausgewählt sein aus Isländischem Moos *(Lichen islandicus),* Eibisch *(Althaea officinalis L.),* Spitzwegerich *(Plantago lanceolata L.),* Malve *(Malva sylvestris L.* und *M. neglecta WALLR.* und andere), Boxhornklee *(Trigonella foenum-graecum L.),* Salep und Quitte *(Cydonia oblonga MILL.).* Erfindungsgemäß bevorzugt sind Isländisches Moos *(Lichen islandicus)* und/ oder Eibisch *(Althaea officinalis L.),* entweder allein oder in Kombination miteinander. Die einzelnen Schleimdrogen bzw. deren Extrakte sind als solche dem Fachmann hinlänglich bekannt. Für weitere Einzelheiten zu Schleimdrogen und ihren Zubereitungen, Wirkungen und Anwendungen kann verwiesen werden auf H. Wagner "Pharmazeutische Biologie - Drogen und ihre Inhaltsstoffe", Gustav Fischer Verlag, Stuttgart/New York, 1985, insbesondere Seiten 280 ff.

Was die erfindungsgemäß gegebenenfalls eingesetzte Schleimdroge anbelangt, so kann diese erfindungsgemäß in Form der Droge selbst, insbesondere in Form zerkleinerter oder pulverisierter Pflanzenteile oder Pflanzenbestandteile, der pharmazeutischen Zusammensetzung zugesetzt sein. Des weiteren besteht die Möglichkeit, daß die Schleimdroge in Form eines Extraktes, insbesondere eines Trockenextraktes, welcher beispielsweise erhältlich ist ausgehend von einem wäßrigen, alkoholischen oder wäßrig-alkoholischen Extraktes der Droge. Die Verwendung eines Extraktes einer Schleimdroge bietet gegenüber der Verwendung der Droge als solcher den entscheidenden Vorteil, daß hohe Konzentrationen der Schleimdroge zur Anwendung kommen, so daß eine besonders gute therapeutische Wirkung erzielt wird.

Was die Menge an gegebenenfalls vorhandenen Schleimdroge(n) und/oder deren Extrakt(en) in der erfindungsgemäßen pharmazeutischen Zusammensetzung anbelangt, so kann die Menge in weiten Bereichen variieren. Im allgemeinen liegt die Menge an Schleimdroge(n) und/oder deren Extrakt(en) im Bereich von 0,1 bis 25 Gew.-%, vorzugsweise 0,5 bis 20 Gew.-%, besonders bevorzugt 1 bis 10 Gew.-%, bezogen auf die pharmazeutische Zusammensetzung, d. h. bezogen auf die feste Dosierungsform. Dennoch kann es anwendungsbedingt gegebenenfalls vorteilhaft oder erforderlich sein, von den vorgenannten Mengenangaben abzuweichen.

Neben den vorgenannten Wirk- und Inhaltsstoffen kann die erfindungsgemäße pharmazeutische Zusammensetzung außerdem noch weitere Wirk- und/oder Inhaltsstoffe enthalten, beispielsweise Verarbeitungshilfsmittel, Aromen und Aromastoffe, Geschmacksstoffe, Farbstoffe, Süßstoffe und Süßungsmittel, Säuerungsmittel, Stabilisatoren und/oder Antiseptika. Die Menge an diesen weiteren Wirk- und/oder Inhaltsstoffen kann in weiten Bereichen variieren; im allgemeinen liegt sie im Bereich von 0,01 bis 10 Gew.-%, insbesondere 0,1 bis 5 Gew.-%, bezogen auf die erfindungsgemäß pharmazeutische Zusammensetzung. Dennoch kann es gegebenenfalls erforderlich sein, von den vorgenannten Mengen abzuweichen, insbesondere wenn dies anwendungsbedingt erforderlich sein sollte.

Im allgemeinen enthält die erfindungsgemäße pharmazeutische Zusammensetzung die Wirk- und/oder Inhaltsstoffe in einer festen Matrix bzw. Masse. Die Wirk- und/oder Inhaltsstoffe sind also in einer Matrix inkorporiert bzw. eingelagert, so daß sie wirksam geschützt und homogen verteilt sind.

Die feste Dosierungsform zum Lutschen und die Inkorporierung der Wirk- und/oder Inhaltsstoffe in eine feste Matrix bzw. Masse bietet eine Reihe von Vorteilen: Zum einen lassen sich auf diese Weise die Wirk- und Inhaltsstoffe besser dosieren, d. h. es wird eine verbesserte Dosiergenauigkeit erreicht. Zum anderen wird dadurch die Applikations- bzw. Einnahmemöglichkeit verbessert, d. h. die Einnahmemöglichkeit für den Patienten ist sozusagen überall gegeben (z. B. unterwegs auf Reisen, im Freien etc.), da keine speziellen Zubereitungsformen angemischt werden müssen. Darüber hinaus liefert die feste Dosierungsform den entscheidenden Vorteil, daß die Wirkstoffe in festen Zubereitungen im allgemeinen lagerstabiler als in Lösungen oder Suspensionen sind. Weiterhin gewährt die feste Dosierungsform eine definierte Verweilzeit bzw. Verweildauer im Mund- und Rachenraum und somit eine effiziente und kontrollierte Therapierung. Schließlich ermöglicht die feste Dosierungsform, insbesondere durch die Einlagerung der Wirk- und Inhaltsstoffe in die Matrix, eine verbesserte Kombination mit anderen Wirkstoffen und deren gleichmäßige, homogene Verteilung über die Matrix.

Als Matrix bzw. Masse für die Einlagerung der Wirk- und Inhaltsstoffe eignen sich insbesondere Zucker aller Art und/oder Zuckeraustauschstoffe aller Art. Beispiele für erfindungsgemäß geeignete Zucker sind beispielsweise Saccharose, Glucose, insbesondere Dextrose, und Fructose sowie deren Mischungen untereinander. Erfindungsgemäß geeignete Zuckeraustauschstoffe sind insbesondere ausgewählt aus Zuckeralkoholen. Erfindungsgemäß bevorzugte Zukkeraustauschstoffe sind ausgewählt aus der Gruppe von Mannit, Xylit, Sorbit, Isomalt, Maltitsirup, Lactit, Leucrose, Fructooligosacchariden, Glucanen und Polyglucose.

Während Zucker und Süßstoffe gemeinsam als Süßungsmittel bezeichnet werden, werden - im Gegensatz zu den intensiv schmeckenden Süßstoffen - Zuckeraustauschstoffe technologisch wie Saccharose eingesetzt, d. h. sie besitzen einen "Körper" und einen physiologischen Brennwert ("nutritive Zukkeraustauschstoffe"). Die Süßkraft entspricht in weiten Grenzen etwa der von Saccharose. Der physiologische Vorteil der Zuckeraustauschstoffe im Vergleich zu Saccharose liegt in der insulinunhabhängigen Metabolisierung (vorteilhaft z. B. für Diabetiker) und in der zum Teil verminderten kariogenen Wirkung. Für einige Zuckeraustauschstoffe (z. B. Xylit) ist eine antikariogene Wirkung beschrieben.

Der Begriff "Zuckeralkohol" ist die Gruppenbezeichnung für die aus Monosacchariden durch Reduktion der Carbonylgruppe entstehenden Polyhydroxyverbindungen, die keine Zucker sind, gleichwohl aber süß schmecken und deshalb gegebenenfalls Verwendung als Zuckeraustauschstoffe finden können. Man unterscheidet bei diesen im allgemeinen kristallinen, wasserlöslichen Polyolen nach der Anzahl der im Molekül enthaltenen Hydroxygruppen sogenannte Tetrite, Pentite, Hexite usw. Natürlich vorkommende Zuckeralkohole sind z. B. Glycerin, Threit und Erythrit, Adonit (Ribit), Arabit (früher: Lyxit) und Xylit, Dulcit (Galactit), Mannit und Sorbit (Glucit).

Für weitergehende Einzelheiten zu den Begriffen "Zucker", "Zuckeraustauschstoffe" und "Zuckeralkohole" kann beispielsweise verwiesen werden auf Römpp Chemie-Lexikon, 10. Auflage, Band 6, Georg Thieme Verlag, Stuttgart/New York, 1999, Seiten 5096 bis 5100, Stichworte: "Zucker", "Zukkeralkohole" und "Zuckeraustauschstoffe".

Die Menge an Matrix bzw. fester Masse, insbesondere an Zuckern und/oder Zuckeraustauschstoffen, kann in weiten Bereichen variieren. Die Menge an Matrixmasse (d. h. Zuckern und/oder Zuckeraustauschstoffen) liegt im allgemeinen im Bereich von 70 bis 99,95 Gew.-%, insbesondere 80 bis 99,95 Gew.-%, bevorzugt 90 bis 99 Gew.-%, besonders

bevorzugt 93 bis 99 Gew.-%, bezogen auf die pharmazeutische Zusammensetzung. Dennoch kann es anwendungsbedingt bzw. fallbezogen gegebenenfalls erforderlich oder vorteilhaft sein, von den vorgenannten Mengen abzuweichen.

Gemäß einer besonders bevorzugten Ausführungsform liegt die erfindungsgemäße pharmazeutische Zusammensetzung bzw. die erfindungsgemäße feste Dosierungsform als Lutschtablette, insbesondere in Form einer Hartkaramelle, vor. Wenn die erfindungsgemäße feste Dosierungsform auf Lutschtablettenbasis gebildet ist, führt dies dazu, daß die feste Dosierungsform beim Lutschen eine therapeutisch wirksame Menge der Wirk- und/oder Inhaltsstoffe freisetzt, wenn die feste Dosierungsform im Mund- und Rachenraum eines Patienten verabreicht und gelutscht wird.

Bei der bevorzugten Ausgestaltung der erfindungsgemäßen pharmazeutischen Zusammensetzung in Form einer Lutschtablette, insbesondere auf Hartkaramellenbasis, kann das Gewicht der Lutschtablette in weiten Bereichen variieren. Im allgemeinen beträgt das Gesamtgewicht der Lutschtablette 0,5 bis 5 g, vorzugsweise 1 bis 4 g.

Gemäß einer besonders bevorzugten Ausgestaltung ist die erfindungsgemäße pharmazeutische Zusammensetzung in Form einer Lutschtablette, insbesondere auf Hartkaramellenbasis, ausgebildet, wobei die Lutschtablette ein Gesamtgewicht von 0,5 bis 5 g, insbesondere 1 bis 4 g, aufweist und je Lutschtablette enthält:

- Octenidin, insbesondere Octenidindihydrochlorid, in Mengen von 0,1 bis 10 mg, insbesondere 0,25 bis 7,5 mg, vorzugsweise 0,5 bis 5 mg;

- gegebenenfalls mindestens ein Lokalanästhetikum in Mengen von 1 bis 55 mg, insbesondere 2,5 bis 30 mg, vorzugsweise 5 bis 20 mg;

- gegebenenfalls mindestens einen weiteren Wirk- und/oder Inhaltsstoff, ausgewählt aus der Gruppe von Verarbeitungshilfsmitteln, Aromen und Aromastoffen, Geschmacksstoffen, Süßstoffen und Süßungsmitteln, Säuerungsmitteln, Stabilisatoren, Antiseptika und/oder Farbstoffen, in Mengen von 5 bis 500 mg, insbesondere 10 bis 250 mg, vorzugsweise 15 bis 100 mg;

- gegebenenfalls mindestens eine Schleimdroge und/oder deren Extrakt in Mengen von 10 bis 250 mg, insbesondere 30 bis 150 mg;

- Rest: Zucker und/oder Zuckeraustauschstoffe als Masse und/oder Matrix.

Die Herstellung der erfindungsgemäßen pharmazeutischen Zusammensetzung erfolgt in an sich bekannter Weise. Für weitergehende diesbezügliche Einzelheiten kann auch auf die nachfolgenden Ausführungsbeispiele verwiesen werden.

Bei der Herstellung von Lutschtabletten auf Hartkaramellenbasis kann beispielsweise derart vorgegangen werden, daß zunächst die Wirk- und Inhaltsstoffe - gegebenenfalls nach Zerkleinerung - eingewogen werden und dann in die zuvor erwärmte Grundsubstanz auf Basis von Zuckern oder Zuckerersatzstoffen (z. B. Isomalt) eingemischt werden, gefolgt von einer Formung von Lutschtabletten und nachfolgendem Abkühlen. Derartige Herstellverfahren sind dem Fachmann als solche wohlbekannt.

Die Herstellung von Hartkaramellen kann typischerweise wie folgt durchgeführt werden: Bei der Herstellung von erfindungsgemäßen Lutschtabletten, insbesondere in Form von Hartkaramellen, werden zunächst der Zucker bzw. die Zuckerersatzstoffe bzw. Zuckeraustauschstoffe in Wasser gelöst, anschließend bei Temperaturen erwärmt (z. B. auf Temperaturen zwischen 120 und 140 °C) und schließlich vakuumiert. Dieser erwärmten bzw. heißen Masse können dann die Wirk- und Inhaltsstoffe, d. h. Octenidin sowie gegebenenfalls weitere Inhaltsstoffe, wie z. B. Lokalanästhetikum, Schleimdrogen bzw. deren Extrakte, Farbstoffe, Aromen, Süßstoffe etc., in fester oder flüssiger Form zudosiert werden. Nach kontrolliertem Abkühlen (z. B. auf Temperaturen unterhalb von etwa 75 °C) können die Hartkaramellen in der gewünschten Form geprägt werden. Die Formen können - wie gewünscht - beispielsweise rund oder polygonal sein. Abschließend können die Hartkaramellen kontrolliert auf Raumtemperatur abgekühlt und sortiert werden. Die Verpakkung der auf diese Weise hergestellten Hartkaramellen kann vereinzelt in Blistern oder Sachets oder auch gesammelt in Beuteln bzw. Tüten erfolgen.

Die erfindungsgemäße Zusammensetzung eignet sich insgesamt somit hervorragend zur prophylaktischen wie kurativen topischen Behandlung von entzündlichen Erkrankungen des Mund- und Rachenraums, insbesondere auch zu Zwecken der Desinfektion des Mund- und Rachenraums, beispielsweise bei Halsschmerzen, Heiserkeit, Katharren, Erkältungserkrankungen, Angina, Entzündungen des Mund- und Rachenraumes sowie des Zahnfleisches. Die erfindungsgemäße pharmazeutische Zusammensetzung ist im Indikationsbereich wirksam sowohl gegen typische Bakterien, wie z. B. Staphylococcus aureus, Escherichia coli, Streptococcus pyogenes, Pseudomonas aeruginosa, Streptococcus pneumoniae, Streptococcus mutans, Streptococcus sanguis und Haemophilus influenzae, als auch gegen typische Tonsillitisviren, wie z. B. Adenoviren und Herpesviren.

Ein weiterer Gegenstand der vorliegenden Erfindung ist die Verwendung der zuvor beschriebenen pharmazeutischen Zusammensetzung nach der vorliegenden Erfindung zur topischen Behandlung von entzündlichen Erkrankungen des Mund-/Rachenraums bzw. zur Herstellung eines Arzneimittels zur topischen Behandlung von entzündlichen Erkrankungen des Mund-/Rachenraums sowie zu Zwecken der Desinfektion des Mund- und Rachenraums, wobei das Octenidin, insbesondere Octenidindehydrochlorid, als pharmazeutische Zusammensetzung in Form einer festen Dosierung zum Lutschen, insbesondere als Lutschtablette, vorzugsweise in Form einer Hartkaramelle, appliziert wird. Für weitergehende Einzelheiten in bezug auf die erfindungsgemäße Verwendung kann auf obige Ausführungen zu der erfindungsgemäßen pharmazeutischen Zusammensetzung verwiesen werden, die in bezug auf die erfindungsgemäße Verwendung entsprechend gelten.

Weitere Ausgestaltungen, Abwandlungen und Variationen sowie Vorteile der vorliegenden Erfindung sind für den Fachmann beim Lesen der Beschreibung ohne weiteres erkennbar und realisierbar, ohne daß er dabei den Rahmen der vorliegenden Erfindung verläßt.

Die folgenden Ausführungsbeispiele dienen lediglich der Veranschaulichung der vorliegenden Erfindung, ohne sie jedoch hierauf zu beschränken.

## AUSFÜHRUNGSBEISPIELE:

### 1. Herstellungsbeispiele:

#### Herstellung erfindungsgemäßer Lutschtabletten auf Hartkaramellenbasis

Es werden verschiedene erfindungsgemäße Lutschtabletten auf Hartkaramellenbasis hergestellt. Für die zukkerhaltige Variante können z. B. Saccharose- und/oder Glucosesirup eingesetzt werden, während für die zuckerfreie Variante Zuckeraustauschstoffe, insbesondere Zuckeralkohole, wie z. B. Maltit (Maltitol) oder Isomalt bzw. Isomaltit (Palatinit®), als Lutschtablettenbasis bzw. Hartkaramellenbasis (Matrix) eingesetzt werden können, wobei sowohl bei der zuckerhaltigen als auch bei der zuckerfreien Variante gegebenenfalls weitere Wirk- und/oder Inhaltsstoffe (z. B. Lokalanästhetika, Schleimdrogen bzw. deren Extrakte, Verarbeitungshilfsmittel, Aromen und Aromastoffe, Geschmacksstoffe, Süßstoffe und Süßungsmittel, Säuerungsmittel, Stabilisatoren, Antiseptika, Farbstoffe etc.) zugesetzt sein können. Die entsprechenden Wirk- und Inhaltsstoffe werden in die Matrix eingearbeitet. Die Herstellung der Lutschtabletten bzw. Hartkaramellen wird dabei wie folgt durchgeführt:

Der Zucker bzw. die Zuckerersatzstoffe/Zuckeraustauschstoffe werden in Wasser gelöst und anschließend bei Temperaturen zwischen 120 und 140 °C gekocht und vakuumiert. Dieser heißen Masse werden der Wirkstoff (Octenidindihydrochlorid) sowie gegebenenfalls weitere Wirk- und/oder Inhaltsstof fe, wie zuvor genannt, in fester oder flüssiger Form zudosiert. Nach kontrolliertem Abkühlen auf Temperaturen unterhalb von 75 °C werden die Hartkaramellen in der gewünschten Form geprägt und nachfolgend verpackt.

Auf diese Weise werden verschiedene Rezepturen erfindungsgemäßer Lutschtabletten mit variablen Mengen an Inhaltsstoffen hergestellt:

Rezeptur 1: Lutschtabletten, zuckerfrei (Isomalt)

| | |
|---|---|
| Octenidindihydrochlorid: | 0,05 - 2,0 Gew.-% |
| Isomalt: | 93,0 - 99,95 Gew.-% |
| Säuerungsmittel, Farbstoffe, Süßstoffe und Aromen: | 0 - 5,0 Gew.-% |
| Summe: | 1.000 - 4.000 mg |

Rezeptur 2: Lutschtablette, zuckerfrei (Maltitol)

| | |
|---|---|
| Octenidindihydrochlorid: | 0,05 - 2,0 Gew.-% |
| Maltitol: | 93,0 - 99,95 Gew.-% |
| Säuerungsmittel, Farbstoffe, Süßstoffe und Aromen: | 0 - 5,0 Gew.-% |
| Summe: | 1.000 - 4.000 mg |

Rezeptur 3: Lutschtabletten, zuckerfrei (Maltitol/Isomalt)

| | |
|---|---|
| Octenidindihydrochlorid: | 0,05 - 2,0 Gew.-% |
| Maltitol/Isomalt 1 : 0,10 - 4: | 93,0 - 99,95 Gew.-% (Trockensubstanz) |

(fortgesetzt)

| | |
|---|---|
| Rezeptur 3: Lutschtabletten, zuckerfrei (Maltitol/Isomalt) | |
| Säuerungsmittel, Farbstoffe, Süßstoffe und Aromen: | 0 - 5,0 Gew.-% |
| Summe: | 1.000 - 4.000 mg |
| Rezeptur 4: Lutschtabletten, zuckerhaltig (Saccharose/Glucose-Sirup) | |
| Octenidindihydrochlorid: | 0,05 - 2,0 Gew.-% |
| Saccharose/Glucose-Sirup 1 : 0,5 - 4: | 93,0 - 99,95 Gew.-% (Trockensubstanz) |
| Säuerungsmittel, Farbstoffe, Süßstoffe und Aromen: | 0 - 5,0 Gew.-% |
| Summe: | 1.000 - 4.000 mg |

## 2. Nachweis auf pharmakologische Wirksamkeit

### Prüfbericht

Quantitativer Suspensionstest zur Bestimmung der bakteriziden Wirkung in Anlehnung an DIN EN 1040

### Durchführung

Bakterizide Wirkung (Verdünnung - Neutralisation)

### Identifizierung der Probe

Octenidindihydrochlorid-Lutschtabletten auf Hartkaramellenbasis

### Prüfbedingungen

| | | |
|---|---|---|
| Testkeime: | Staphylococcus (S.) aureus | ATCC 6538 |
| | Escherichia (E.) coli | ATCC 10536 |
| | Streptococcus (Str.) pyogenes | ATCC 12344 |
| | Pseudomonas (P.) aeruginosa | ATCC 15442 |
| | Streptococcus (Str.) pneumoniae | ATCC 33400 |
| | Streptococcus (Str.) mutans | ATCC 25175 |
| | Streptococcus (Str.) sanguis | ATCC 10556 |
| | Haemophilus (H.) influenzae | ATCC 49247 |
| Aussehen des Produkts: | lachsfarbene Lutschtabletten | |
| Probenaufbereitung: | 2,6 g Lutschtabletten wurden in 10 ml künstlichem Speichel (siehe unten) bei 37 °C im Schüttelwasserbad gelöst. | |
| Künstlicher Speichel: | Prüflösung 1 nach 82.10-1 bestehend aus: | |
| | $NaHCO_3$, zur Analyse | 4,2 g |
| | NaCl, zur Analyse | 0,5 g |
| | $K_2CO_3$, zur Analyse | 0,2 g |
| | Aqua dest. | 1.000 ml |
| | Amtliche Sammlung von Untersuchungsverfahren nach § 35 LMBG | |
| Prüftemperatur: | 20 °C ± 1 °C | |
| Einwirkzeit: | 15 min ± 10 sec | |
| Zählverfahren: | Oberflächenverfahren | |
| Bebrütungsdauer: | 48 Stunden, 37 °C ± 1 °C | |

### Prüfverfahren und seine Validierung

| | |
|---|---|
| **Verfahren:** | Verdünnung - Neutralisation |
| **Inaktivierungskombination:** | 3,0 % Tween 80 + 3,0 % Saponin + 0,1 % Histidin + 0,1 % Cystein in Caseinpepton-Sojamehlpepton-Bouillon |

**Prüfergebnisse:**

siehe nachfolgende Tabelle 1

**Schlußfolgerung:**

Mit einer Testkonzentration von 2,6 g / 10 ml künstlichem Speichel erreichten die erfindungsgemäßen Octenidindihydrochlorid-Lutschtabletten auf Hartkaramellenbasis nach einer Einwirkzeit von 15 Minuten die nach DIN EN 1040 geforderte Keimreduktion von > 5,0 log-Stufen gegenüber den Testkeimen E. coli, P. aeruginosa, Str. mutans, Str. sanguis und H. influenzae.

Mit einer Testkonzentration von 2,6 g / 10 ml künstlichem Speichel erreichten die erfindungsgemäßen Octenidindihydrochlorid-Lutschtabletten nach einer Einwirkzeit von 15 Minuten die maximal erreichbare Keimreduktion von > 4,0 log-Stufen gegenüber den Testkeimen S. aureus, Str. pyogenes und Str. pneumoniae.

**Tabelle 1**

| **Prüfkeim** | **Validierungsprüfung** | | | | **Test** | |
|---|---|---|---|---|---|---|
| | Bakteriensuspension | Kontrolle der Toxizität der Inaktivierungskombination | Kontrolle der Inaktivierung | Bakterienprüfsuspension | Verdünnungsstufe des Prüfneutralisationsgemisches | Einwirkzeit 15 min |
| **S. aureus ATCC 6538** | Vc: 284<br>Nv:$2{,}8\times10^{3}$ | Vc: 200/216<br>B=$2{,}1 \times 10^{2}$ | Vc: -/-°<br>C=-* | Vc: 284<br>N=$2{,}8 \times 10^{8}$ | $10^{0}$ | Vc: 0/0 |
| | | | Vc: 276/296<br>C = $2{,}9 \times 10^{2}$ | | $10^{-1}$ | Vc: 0<br>Na: <$1.5\times10^{3}$ R: >$1{,}9\times10^{4}$ = > 4,28 log |
| | | | Vc: 300/352<br>C=$3{,}3 \times 10^{2}$ | | $10^{-2}$ | Vc: 0 |
| | | | Vc: 236/238<br>C = $2{,}4 \times 10^{2}$ | | $10^{-3}$ | Vc: 0 |
| **E. coli ATCC 10536** | Vc: 250<br>Nv = $2{,}5\times10^{3}$ | Vc: 308/268<br>B = $2{,}9 \times 10^{7}$ | Vc: 214/246<br>C = $2{,}3 \times 10^{2}$ | Vc: 250<br>N = $2{,}5 \times 10^{8}$ | $10^{0}$ | Vc: 0/0<br>Na: <$1{,}5\times10^{2}$<br>R: > $1{,}7\times10^{5}$<br>= > 5,23 log |
| | | | | | $10^{-2}$ | Vc: 0 |
| | | | | | $10^{-3}$ | Vc: 0 |
| **Str. pyogenes ATCC 12344** | Vc: 236<br>Nv = $2{,}4 \times 10^{3}$ | Vc: 268/284<br>B = $2{,}8 \times 10^{2}$ | Vc: -/-*<br>C = -* | Vc: 236<br>N = $2{,}4 \times 10^{8}$ | $10^{0}$ | Vc: 0/0 |

(fortgesetzt)

| Prüfkeim | Validierungsprüfung | | | | Test | |
|---|---|---|---|---|---|---|
| | Bakteriensuspension | Kontrolle der Toxizität der Inaktivierungskombination | Kontrolle der Inaktivierung | Bakterienprüfsuspension | Verdünnungsstufe des Prüfneutralisationsgemisches | Einwirkzeit 15 min |
| | | | Vc: 300/328<br>$C=3,1 \times 10^2$ | | $10^{-1}$ | Vc: 0<br>Na: $<1,5x10^3$<br>R:$>1,6x10^4$<br>= > 4,20 log |
| | | | Vc: 288/276 Vc: 288/276<br>$C = 2,8 x 10^2$ | | $10^{-2}$ | Vc: 0 |
| | | | Vc: 272/244<br>$C = 2.6 x 10^2$ | | $10^{-3}$ | Vc: 0 |
| **P. aerugniosa ATCC 15442** | Vc: 200<br>$Nv = 2,0 x 10^3$ | Vc: 216/252<br>$B = 2,3 x 10^2$ | Vc: 292/276<br>$C = 2,8 x 10^2$ | Vc: 200<br>$N = 2,0 x 10^8$ | $10^0$ | Vc: 0/0<br>Na: $<1,5 \times 10^2$<br>R:$>1,3x10^5$<br>= > 5,11 log |
| | | | | | $10^{-2}$ | Vc: 0 |
| | | | | | $10^{-3}$ | Vc: 0 |
| **Str. pneumoniae ATCC 33400** | Vc: 288<br>Nv: = $2,9 x 10^3$ | Vc: 284/348<br>$B = 3,2 x 10^2$ | Vc: 48/76*<br>C = -* | Vc: 288<br>$N = 3,0 x 10^8$ | 10° | Vc: 0/0 |
| | | | Vc: 200/226<br>$C = 2,1 \times 10^2$ | | $10^{-1}$ | Vc: 0<br>Na: $<1,5x10^2$<br>R:$>1,9x10^4$<br>= > 4,28 log |
| | | | Vc: 232/272<br>$C = 2,5 \times 10^2$ | | $10^{-2}$ | Vc: 0 |
| | | | Vc: 328/264<br>$C = 3,0 \times 10^2$ | | $10^{-3}$ | Vc: 0 |
| **Str. mutans ATCC 25175** | Vc: 240 | Vc: 284/292 | Vc: 252/246 | Vc: 240 | 10° | Vc: 0/0<br>Na: $<1,5x10^2$ |

(fortgesetzt)

| Prüfkeim | Validierungsprüfung | | | | Test | |
|---|---|---|---|---|---|---|
| | Bakteriensuspension | Kontrolle der Toxizität der Inaktivierungskombination | Kontrolle der Inaktivierung | Bakterienprüfsuspension | Verdünnungsstufe des Prüfneutralisationsgemisches | Einwirkzeit 15 min |
| | Nv = 2,4 x $10^3$ | B = 2,9 x $10^2$ | C = 2,5 x $10^2$ | N = 2,4 x $10^8$ | | R:>1,6x$10^3$<br>= > 5,20 log |
| | | | | | $10^{-2}$ | Vc: 0 |
| | | | | | $10^{-3}$ | Vc: 0 |
| **Str. sanguis ATCC 10556** | Vc: 180<br>Nv = 1,8 x $10^3$ | Vc: 210/222<br>B = 2,2 x $10^2$ | Vc: 204/Z50<br>C = 2.3 x $10^2$ | Vc: 180<br>N = 1,8 x $10^8$ | 10° | Vc: 0/0<br>Na: <1,5x$10^2$<br>R: >1,2x$10^5$<br>= > 5,08 log |
| | | | | | $10^{-2}$ | Vc: 0 |
| | | | | | $10^{-3}$ | Vc: 0 |
| **H. influenzae ATCC 49247** | Vc: 214<br>Nv = 2,1 x $10^3$ | Vc: 160/172<br>B = 1,7 x $10^2$ | Vc: 186/180<br>C = 1,8 x $10^2$ | Vc: 214<br>N = 2,1 x $10^8$ | $10^0$ | Vc: 0/0<br>Na: <1,5x$10^2$<br>R:>1,4x$10^5$<br>=>5,15 log |
| | | | | | $10^{-2}$ | Vc: 0 |
| | | | | | $10^{-3}$ | Vc: 0 |

Erläuterungen zu Tabelle 1:
Vc = Lebendkeimzahl
N = Zahl der KBE/ml der Bakterienprüfsuspension
Nv = Zahl der KBE/ml der verdünnten Bakterienprüfsuspension (= Bakteriensuspension)
Na = Anzahl der KBE je ml im Prüfgemisch
B = durchschnittliche Zahl der KBE/ml des Gemisches Bakterien + Inaktivierungskombination (Kontrolle Toxizität)
C = durchschnittliche Zahl der KBE/ml im Kontrollversuch für die Verdünnung - Neutralisation (= Kontrolle Inaktivierung)
* = Hinweis auf mangelnde Inaktivierung

R = Verminderung der Lebendkeimzahl, $\frac{N \times 10^{-1}}{Na}$

**Patentansprüche**

1. Pharmazeutische Zusammensetzung in Form einer festen Dosierung zum Lutschen, insbesondere Lutschtablette, zur topischen Behandlung von entzündlichen Erkrankungen des Mund- und Rachenraums, wobei die Zusammensetzung eine therapeutisch wirksame Menge an Octenidin enthält.

2. Pharmazeutische Zusammensetzung nach Anspruch 1, enthaltend das Octenidin in Form des Octenidindihydrochlorids.

3. Pharmazeutische Zusammensetzung nach Anspruch 1 oder 2, enthaltend Octenidin, insbesondere Octenidindihydrochlorid, in Mengen von 0,01 bis 5 Gew.-%, insbesondere 0,025 bis 3 Gew.-%, vorzugsweise 0,05 bis 2,5 Gew.-%, bezogen auf die pharmazeutische Zusammensetzung.

4. Pharmazeutische Zusammensetzung nach einem oder mehreren der vorangehenden Ansprüche, enthaltend außerdem mindestens ein Lokalanästhetikum, insbesondere in Mengen von 0,01 bis 5 Gew.-%, insbesondere 0,05 bis 4 Gew.-%, vorzugsweise 0,1 bis 2,5 Gew.-%, besonders bevorzugt 0,2 bis 0,6 Gew.-%, bezogen auf die pharmazeutische Zusammensetzung.

5. Pharmazeutische Zusammensetzung nach Anspruch 4, wobei das Lokalanästhetikum ein Lokalanästhetikum auf Basis eines organischen Säureesters oder Säureamids, vorzugsweise Säureesters, ist und/oder wobei das Lokalanästhetikum aus der Gruppe von Lokalanästhetika vom Estertyp oder Amidtyp, vorzugsweise vom Estertyp, ausgewählt ist.

6. Pharmazeutische Zusammensetzung nach Anspruch 4 oder 5, wobei das Lokalanästhetikum ausgewählt ist aus der Gruppe von Benzocain, Procain, Tetracain, Lidocain, Etidocain, Prilocain, Mepivacain, Bupivacain, S-Ropivacain und Articain, vorzugsweise Benzocain.

7. Pharmazeutische Zusammensetzung nach einem oder mehreren der Ansprüche 4 bis 6, wobei das Lokalanästhetikum Benzocain ist.

8. Pharmazeutische Zusammensetzung nach einem oder mehreren der vorangehenden Ansprüche, enthaltend außerdem mindestens eine Schleimdroge und/oder deren Extrakt, insbesondere in Mengen von 0,1 bis 25 Gew.-%, vorzugsweise 0,5 bis 20 Gew.-%, besonders bevorzugt 1 bis 10 Gew.-%, bezogen auf die pharmazeutische Zusammensetzung.

9. Pharmazeutische Zusammensetzung nach Anspruch 8, wobei die Schleimdroge in Form der Droge, insbesondere in Form zerkleinerter oder pulverisierter Pflanzenteile oder Pflanzenbestandteile, zugesetzt ist.

10. Pharmazeutische Zusammensetzung nach Anspruch 8, wobei die Schleimdroge in Form eines Extraktes zugesetzt ist, insbesondere in Form eines Trockenextraktes.

11. Pharmazeutische Zusammensetzung nach einem oder mehreren der Ansprüche 8 bis 10, wobei die Schleimdroge ausgewählt ist aus Isländischem Moos *(Lichen islandicus),* Eibisch *(Althaea officinalis L.),* Spitzwegerich *(Plantago lanceolata* L.), Malve *(Malva sylvestris L.* und *M. neglecta WALLR.* und andere), Boxhornklee *(Trigonella foenum-graecum L.),* Salep und Quitte *(Cydonia oblonga MILL.*).

12. Pharmazeutische Zusammensetzung nach einem oder mehreren der vorangehenden Ansprüche, enthaltend außerdem weitere Wirk- und/oder Inhaltsstoffe, insbesondere ausgewählt aus der Gruppe von Verarbeitungshilfsmitteln, Aromen und Aromastoffen, Geschmacksstoffen, Süßstoffen und Süßungsmitteln, Säuerungsmitteln, Stabilisatoren, Antiseptika und/oder Farbstoffen.

13. Pharmazeutische Zusammensetzung nach Anspruch 12, enthaltend die weiteren Wirk- und/oder Inhaltsstoffe in Mengen von insgesamt 0,01 bis 10 Gew.-%, vorzugsweise 0,1 bis 5 Gew.-%, bezogen auf die pharmazeutische Zusammensetzung.

14. Pharmazeutische Zusammensetzung nach einem oder mehreren der vorangehenden Ansprüche, enthaltend die Wirk- und/oder Inhaltsstoffe in einer festen Matrix und/oder Masse.

**15.** Pharmazeutische Zusammensetzung nach einem oder mehreren der vorangehenden Ansprüche, enthaltend die Wirk- und/oder Inhaltsstoffe in einer Matrix und/oder Masse auf Basis von Zuckern und/oder Zuckeraustauschstoffen.

**16.** Pharmazeutische Zusammensetzung nach Anspruch 15, wobei die Zucker ausgewählt sind aus der Gruppe von Saccharose, Glucose, insbesondere Dextrose, und Fructose und/oder wobei die Zuckeraustauschstoffe ausgewählt sind aus Zuckeralkoholen und/oder aus der Gruppe von Mannit, Xylit, Sorbit, Isomalt, Maltitsirup, Lactit, Leucrose, Fructooligosacchariden, Glucanen und Polyglucose.

**17.** Pharmazeutische Zusammensetzung nach Anspruch 15 oder 16, enthaltend Zukker und/oder Zuckeraustauschstoffe in Mengen von 70 bis 99,95 Gew.-%, insbesondere 80 bis 99,95 Gew.-%, vorzugsweise 90 bis 99 Gew.-%, besonders bevorzugt 93 bis 99 Gew.-%, bezogen auf die pharmazeutische Zusammensetzung.

**18.** Pharmazeutische Zusammensetzung nach einem oder mehreren der vorangehenden Ansprüche, wobei die feste Dosierungsform eine Lutschtablette ist, insbesondere in Form einer Hartkaramelle, und/oder wobei die feste Dosierungsform auf Lutschtablettenbasis gebildet ist, so daß die feste Dosierungsform eine therapeutisch wirksame Menge der Wirk- und/oder Inhaltsstoffe beim Lutschen freisetzt, wenn die feste Dosierungsform im Mund- und Rachenraum eines Patienten verabreicht und gelutscht wird.

**19.** Pharmazeutische Zusammensetzung nach einem oder mehreren der vorangehenden Ansprüche, insbesondere zur topischen Behandlung von entzündlichen Erkrankungen des Mund- und Rachenraums, auf Lutschtablettenbasis, wobei die feste Dosierungsform Octenidindihydrochlorid, gegebenenfalls zusammen mit mindestens einem Lokalanästhetikum, insbesondere Benzocain oder Lidocain, enthält.

**20.** Pharmazeutische Zusammensetzung nach einem oder mehreren der vorangehenden Ansprüche, wobei die pharmazeutische Zusammensetzung in Form einer Lutschtablette, insbesondere auf Hartkaramellenbasis, vorliegt, wobei die Lutschtablette ein Gesamtgewicht von 0,5 bis 5 g, insbesondere 1 bis 4 g, auf weist, enthaltend je Lutschtablette:

- Octenidin, insbesondere Octenidindihydrochlorid, in Mengen von 0,1 bis 10 mg, insbesondere 0,25 bis 7,5 mg, vorzugsweise 0,5 bis 5 mg;
- gegebenenfalls mindestens ein Lokalanästhetikum in Mengen von 1 bis 55 mg, insbesondere 2,5 bis 30 mg, vorzugsweise 5 bis 20 mg;
- gegebenenfalls mindestens einen weiteren Wirk- und/oder Inhaltsstoff, ausgewählt aus der Gruppe von Verarbeitungshilfsmitteln, Aromen und Aromastoffen, Geschmacksstoffen, Süßstoffen und Süßungsmitteln, Säuerungsmitteln, Stabilisatoren, Antiseptika und/oder Farbstoffen, in Mengen von 5 bis 500 mg, insbesondere 10 bis 250 mg, vorzugsweise 15 bis 100 mg;
- gegebenenfalls mindestens Schleimdroge und/oder deren Extrakt in Mengen von 10 bis 250 mg, insbesondere 30 bis 150 mg;
- Rest: Zucker und/oder Zuckeraustauschstoffe als Masse und/oder Matrix.

**21.** Verwendung von Octenidin, insbesondere in Form des Octenidindihydrochlorids, zur Herstellung eines Arzneimittels zur topischen Behandlung von entzündlichen Erkrankungen des Mund- und Rachenraums und/oder zu Zwecken der Desinfektion des Mund- und Rachenraums, wobei das Octenidin, insbesondere Octenidindihydrochlorid, als pharmazeutische Zusammensetzung in Form einer festen Dosierung zum Lutschen, insbesondere als Lutschtablette, vorzugsweise in Form einer Hartkaramelle, appliziert wird.

**22.** Verwendung nach Anspruch 21, wobei die feste Dosierungsform, insbesondere Lutschtablette, eine therapeutisch wirksame Menge der Wirk- und/oder Inhaltsstoffe beim Lutschen freisetzt, wenn die feste Dosierungsform im Mund- und Rachenraum eines Patienten verabreicht und gelutscht wird.

**23.** Verwendung nach Anspruch 21 oder 22, wobei das Octenidin, insbesondere Octenidindihydrochlorid, in Kombination mit mindestens einem Lokalanästhetikum und/oder in Kombination mit mindestens einer Schleimdroge und/oder deren Extrakt eingesetzt wird.

**24.** Verwendung nach einem der Ansprüche 21 bis 23, wobei das Octenidin, insbesondere Octenidindihydrochlorid, in Dosen von 0,1 bis 10 mg, insbesondere 0,25 bis 7,5 mg, vorzugsweise 0,5 bis 5 mg, pro Dosiereinheit, insbesondere pro Lutschtablette, verabreicht wird.

## Claims

1. Pharmaceutical composition in the form of a solid dose for sucking, in particular a lozenge, for the topical treatment of inflammatory diseases of the oral and pharyngeal cavity, where the composition comprises a therapeutically efficacious amount of octenidine.

2. Pharmaceutical composition according to Claim 1, comprising the octenidine in the form of octenidine dihydrochloride.

3. Pharmaceutical composition according to Claim 1 or 2, comprising octenidine, in particular octenidine dihydrochloride, in amounts of from 0.01 to 5% by weight, in particular 0.025 to 3% by weight, preferably 0.05 to 2.5% by weight, based on the pharmaceutical composition.

4. Pharmaceutical composition according to one or more of the preceding claims, additionally comprising a least one local anaesthetic, in particular in amounts of from 0.01 to 5% by weight, in particular 0.05 to 4% by weight, preferably 0.1 to 2.5% by weight, particularly preferably 0.2 to 0.6% by weight, based on the pharmaceutical composition.

5. Pharmaceutical composition according to Claim 4, where the local anaesthetic is a local anaesthetic based on an organic acid ester or acid amide, preferably acid ester, and/or where the local anaesthetic is selected from the group consisting of local anaesthetics of the ester type or amide type, preferably of the ester type.

6. Pharmaceutical composition according to Claim 4 or 5, where the local anaesthetic is selected from the group consisting of benzocaine, procaine, tetracaine, lidocaine, etidocaine, prilocaine, mepivacaine, bupivacaine, S-ropivacaine and articaine, preferably benzocaine.

7. Pharmaceutical composition according to one or more of Claims 4 to 6, where the local anaesthetic is benzocaine.

8. Pharmaceutical composition according to one or more of the preceding claims, additionally comprising at least one mucilaginous drug and/or its extract, in particular in amounts of from 0.1 to 25% by weight, preferably 0.5 to 20% by weight, particularly preferably 1 to 10% by weight, based on the pharmaceutical composition.

9. Pharmaceutical composition according to Claim 8, where the mucilaginous drug is added in the form of the drug, in particular in the form of comminuted or pulverized plant parts or plant constituents.

10. Pharmaceutical composition according to Claim 8, where the mucilaginous drug is added in the form of an extract, in particular in the form of a dry extract.

11. Pharmaceutical composition according to one or more of Claims 8 to 10, where the mucilaginous drug is selected from Icelandic moss *(Lichen islandicus),* marshmallow *(Althaea officinalis L.)* ribwort *(Plantago lanceolata L. )* mallow *(Malva sylvestris L.* and *M neglecta WALLR.* and others), fenugreek *(Trigonella foenum-graecum L.),* salep and quince *(Cydonia oblonga MILL.).*

12. Pharmaceutical composition according to one or more of the preceding claims, additionally comprising further active substances and/or constituents, in particular selected from the group consisting of processing aids, flavours and flavouring substances, flavour additives, sweeteners and sweetenings, acidifying agents, stabilizers, antiseptics and/or colourants.

13. Pharmaceutical composition according to Claim 12, comprising the further active substances and/or constituents in amounts of altogether 0.01 to 10% by weight, preferably 0.1 to 5% by weight, based on the pharmaceutical composition.

14. Pharmaceutical composition according to one or more of the preceding claims, comprising the active substances and/or constituents in a solid matrix and/or mass.

15. Pharmaceutical composition according to one or more of the preceding claims, comprising the active substances and/or constituents in a matrix and/or mass based on sugars and/or sugar substitutes.

16. Pharmaceutical composition according to Claim 15, where the sugars are selected from the group consisting of sucrose, glucose, in particular dextrose, and fructose and/or where the sugar substitutes are selected from sugar

alcohols and/or from the group consisting of mannitol, xylitol, sorbitol, isomalt, maltitol syrup, lactitol, leucrose, fructooligosaccharides, glucans and polyglucose.

**17.** Pharmaceutical composition according to Claim 15 or 16, comprising sugars and/or sugar substitutes in amounts of from 70 to 99.95% by weight, in particular 80 to 99.95% by weight, preferably 90 to 99% by weight, particularly preferably 93 to 99% by weight, based on the pharmaceutical composition.

**18.** Pharmaceutical composition according to one or more of the preceding claims, where the solid dose form is a lozenge, in particular in the form of a hard caramel, and/or where the solid dose form is formed based on a lozenge, such that the solid dose form releases a therapeutically efficacious amount of the active substances and/or constituents on sucking if the solid dose form is administered to and sucked in the oral and pharyngeal cavity of a patient.

**19.** Pharmaceutical composition according to one or more of the preceding claims, in particular for the topical treatment of inflammatory diseases of the oral and pharyngeal cavity, based on a lozenge, where the solid dose form comprises octenidine dihydrochloride, optionally together with a least one local anaesthetic, in particular benzocaine or lidocaine.

**20.** Pharmaceutical composition according to one or more of the preceding claims, where the pharmaceutical composition is present in the form of a lozenge, in particular based on a hard caramel, where the lozenge has a total weight of 0.5 to 5 g, in particular 1 to 4 g, comprising per lozenge:

- octenidine, in particular octenidine dihydro-chloride, in amounts of from 0.1 to 10 mg, in particular 0.25 to 7.5 mg, preferably 0.5 to 5 mg;
- optionally a least one local anaesthetic in amounts of from 1 to 55 mg, in particular 2.5 to 30 mg, preferably 5 to 20 mg;
- optionally a least one further active substance and/or constituent, selected from the group consisting of processing aids, flavours and flavouring substances, flavour additives, sweeteners and sweetenings, acidifying agents, stabilizers, antiseptics and/or colourants, in amounts of from 5 to 500 mg, in particular 10 to 250 mg, preferably 15 to 100 mg;
- optionally at least one mucilaginous drug and/or its extract in amounts of from 10 to 250 mg, in particular 30 to 150 mg;
- remainder: sugar and/or sugar substitutes as a mass and/or matrix.

**21.** Use of octenidine, in particular in the form of octenidine dihydrochloride, for the production of a medicament for the topical treatment of inflammatory diseases of the oral and pharyngeal cavity and/or for purposes of the disinfection of the oral and pharyngeal cavity, where the octenidine, in particular octenidine dihydro-chloride, is administered as a pharmaceutical composition in the form of a solid dose for sucking, in particular as a lozenge, preferably in the form of a hard caramel.

**22.** Use according to Claim 21, where the solid dose form, in particular a lozenge, releases a therapeutically efficacious amount of the active substances and/or constituents on sucking if the solid dose form is administered to and sucked in the oral and pharyngeal cavity of a patient.

**23.** Use according to Claim 21 or 22, where the octenidine, in particular octenidine dihydro-chloride, is employed in combination with a least one local anaesthetic and/or in combination with at least one mucilaginous drug and/or its extract.

**24.** Use according to one of Claims 21 to 23, where the octenidine, in particular octenidine dihydro-chloride, is administered in doses of from 0.1 to 10 mg, in particular 0.25 to 7.5 mg, preferably 0.5 to 5 mg, per dose unit, in particular per lozenge.

## Revendications

**1.** Composition pharmaceutique sous forme d'une forme galénique solide à sucer, en particulier d'un comprimé à sucer, destinée au traitement topique de maladies inflammatoires de la cavité bucco-pharyngée, la composition contenant une quantité thérapeutiquement efficace d'octénidine.

**2.** Composition pharmaceutique selon la revendication 1, contenant l'octénidine sous forme du dichlorhydrate d'octénidine.

3. Composition pharmaceutique selon la revendication 1 ou 2, contenant de l'octénidine, en particulier du dichlorhydrate d'octénidine, en quantités de 0,01 à 5 % en poids, en particulier de 0,025 à 3 % en poids, de préférence de 0,05 à 2,5 % en poids, par rapport à la composition pharmaceutique.

4. Composition pharmaceutique selon une ou plusieurs des revendications précédentes, contenant en outre au moins un anesthésique local, en particulier en quantités de 0,01 à 5 % en poids, en particulier de 0,05 à 4 % en poids, de préférence de 0,1 à 2,5 % en poids, de façon particulièrement préférée, de 0,2 à 0,6 % en poids, par rapport à la composition pharmaceutique.

5. Composition pharmaceutique selon la revendication 4, dans laquelle l'anesthésique local est un anesthésique local à base d'un amide ou ester d'acide organique, de préférence d'un ester d'acide, et/ou dans laquelle l'anesthésique local est choisi dans le groupe des anesthésiques locaux du type ester ou du type amide ; de préférence du type ester.

6. Composition pharmaceutique selon la revendication 4 ou 5, dans laquelle l'anesthésique local est choisi dans l'ensemble constitué par la benzocaïne, la procaïne, la tétracaïne, la lidocaïne, l'étidocaïne, la prilocaïne, la mépivacaïne, la bupivacaïne, la S-ropivacaïne et l'articaïne, de préférence est la benzocaïne.

7. Composition pharmaceutique selon une ou plusieurs des revendications 4 à 6, dans laquelle l'anesthésique local est la benzocaïne.

8. Composition pharmaceutique selon une ou plusieurs des revendications précédentes, contenant en outre au moins un produit mucilagineux et/ou un extrait de celui-ci, en particulier en quantités de 0,1 à 25 % en poids, de préférence de 0,5 à 20 % en poids, de façon particulièrement préférée, de 1 à 10 % en poids, par rapport à la composition pharmaceutique.

9. Composition pharmaceutique selon la revendication 8, dans laquelle le produit mucilagineux est ajouté sous forme du produit, en particulier sous forme de parties de plantes ou de composants végétaux, fragmentés ou pulvérisés.

10. Composition pharmaceutique selon la revendication 8, dans laquelle le produit mucilagineux est ajouté sous forme d'un extrait, en particulier sous forme d'un extrait sec.

11. Composition pharmaceutique selon une ou plusieurs des revendications 8 à 10, dans laquelle le produit mucilagineux est choisi parmi la mousse d'Islande *(Lichen islandicus),* la guimauve officinale *(Althaea officinalis L.),* le plantain lancéolé *(Plantago lanceolata L.),* la mauve sylvestre *(Malva silvestris L.* et *M neglecta WALLR.* et autres), le fénugrec *(Trigonella foenum-graecum L.),* le salep et le coing *(Cydonia oblonga MILL.).*

12. Composition pharmaceutique selon une ou plusieurs des revendications précédentes, contenant en outre d'autres principes actifs et/ou composants, en particulier choisis dans l'ensemble constitué par des adjuvants de fabrication, des arômes et des substances aromatisantes, des agents de sapidité, des édulcorants et des agents sucrants, des acidifiants, des stabilisants, des antiseptiques et/ou des colorants.

13. Composition pharmaceutique selon la revendication 12, contenant les autres principes actifs et/ou composants en quantités allant au total de 0,01 à 10 % en poids, de préférence de 0,1 à 5 % en poids, par rapport à la composition pharmaceutique.

14. Composition pharmaceutique selon une ou plusieurs des revendications précédentes, contenant les principes actifs et/ou les composants dans une matrice et/ou matière solide.

15. Composition pharmaceutique selon une ou plusieurs des revendications précédentes, contenant les principes actifs et/ou les composants dans une matrice et/ou matière à base de sucres et/ou de succédanés de sucre.

16. Composition pharmaceutique selon la revendication 15, dans laquelle les sucres sont choisis dans l'ensemble constitué par le saccharose, le glucose, en particulier le D-glucose, et le fructose et/ou dans laquelle les succédanés de sucre sont choisis parmi des alcools dérivés de sucres et/ou dans le groupe constitué par le mannitol, le xylitol, le sorbitol, l'isomalt, le sirop de maltitol, le lactitol, le leucrose, les fructo-oligosaccharides, les glucanes et le polyglucose.

**17.** Composition pharmaceutique selon la revendication 15 ou 16, contenant des sucres et/ou des succédanés de sucre en quantités de 70 à 99,95 % en poids, en particulier de 80 à 99,95 % en poids, de préférence de 90 à 99 % en poids, de façon particulièrement préférée, de 93 à 99 % en poids, par rapport à la composition pharmaceutique.

**18.** Composition pharmaceutique selon une ou plusieurs des revendications précédentes, dans laquelle la forme galénique solide est un comprimé à sucer, en particulier sous forme d'un caramel dur, et/ou dans laquelle la forme galénique solide est constituée à base de comprimés à sucer, de sorte que la forme galénique solide libère lorsqu'elle est sucée une quantité thérapeutiquement efficace des principes actifs et/ou des composants, lorsque la forme galénique solide est administrée dans la cavité bucco-pharyngée d'un patient, et sucée.

**19.** Composition pharmaceutique selon une ou plusieurs des revendications précédentes, en particulier pour le traitement topique de maladies inflammatoires de la cavité bucco-pharyngée, à base de comprimé à sucer, dans laquelle la forme galénique solide contient du dichlorhydrate d'octénidine, éventuellement conjointement avec au moins un anesthésique local, en particulier de la benzocaïne ou de la lidocaïne.

**20.** Composition pharmaceutique selon une ou plusieurs des revendications précédentes, la composition pharmaceutique se trouvent sous forme d'un comprimé à sucer, en particulier à base de caramel dur, le comprimé à sucer ayant un poids total de 0,5 à 5 g, en particulier de 1 à 4 g, contenant par comprimé à sucer :

- de l'octénidine, en particulier du dichlorhydrate d'octénidine, en quantités de 0,1 à 10 mg, en particulier de 0,25 à 7,5 mg, de préférence de 0,5 à 5 mg ;
- éventuellement au moins un anesthésique local en quantités de 1 à 55 mg, en particulier de 2,5 à 30 mg, de préférence de 5 à 20 mg ;
- éventuellement au moins un autre principe actif et/ou composant, choisi dans le groupe des adjuvants de fabrication, des arômes et des substances aromatisantes, des agents de sapidité, des édulcorants et des agents sucrants, des acidifiants, des stabilisants, des antiseptiques et/ou des colorants, en quantités de 5 à 500 mg, en particulier de 10 à 250 mg, de préférence, de 15 à 100 mg ;
- éventuellement au moins un produit mucilagineux et/ou un extrait de celui-ci, en quantités de 10 à 250 mg, en particulier de 30 à 150 mg ;
- reste : sucres et/ou succédanés de sucre, en tant que matière et/ou matrice.

**21.** Utilisation d'octénidine, en particulier sous forme du dichlorhydrate d'octénidine, pour la fabrication d'un médicament destiné au traitement topique de maladies inflammatoires de la cavité bucco-pharyngée et/ou aux fins de la désinfection de la cavité bucco-pharyngée, dans laquelle l'octénidine, en particulier le dichlorhydrate d'octénidine, est administrée en tant que composition pharmaceutique sous forme d'une forme galénique solide à sucer, en particulier sous forme de comprimé à sucer, de préférence sous forme d'un caramel dur.

**22.** Utilisation selon la revendication 21, dans laquelle la forme galénique solide, en particulier le comprimé à sucer, libère lorsqu'elle est sucée une quantité thérapeutiquement efficace des principes actifs et/ou composants, lorsque la forme galénique solide est administrée dans la cavité bucco-pharyngée d'un patient, et sucée.

**23.** Utilisation selon la revendication 21 ou 22, dans laquelle l'octénidine, en particulier le dichlorhydrate d'octénidine, est utilisée en association avec au moins un anesthésique local et/ou en association avec au moins un produit mucilagineux et/ou un extrait de celui-ci.

**24.** Utilisation selon l'une quelconque des revendications 21 à 23, dans laquelle l'octénidine, en particulier le dichlorhydrate d'octénidine, est administrée à des doses de 0,1 à 10 mg, en particulier de 0,25 à 7,5 mg, de préférence de 0,5 à 5 mg, par dose unitaire, en particulier par comprimé à sucer.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- DE 2708331 C2
- WO 03067988 A1
- EP 0144960 A2
- WO 8705501 A1
- WO 9316681 A1
- DE 10026716 A1


### In der Beschreibung aufgeführte Nicht-Patentliteratur


- **E. MUTSCHLER et al.** Mutschler Arzneimittelwirkungen - Lehrbuch der Pharmakologie und Toxikologie. Wissenschaftliche Verlagsgesellschaft mbH, 2001, 267 ff
- Römpp Chemie-Lexikon. Georg Thieme Verlag, 1997, vol. 3, 2442
- Römpp Chemie-Lexikon. Georg Thieme Verlag, 1999, vol. 6, 5096-5100